Europäisches Patentamt

European Patent Office

Office européen des brevets

(11) Publication number: **0 636 631 A1**

## EUROPEAN PATENT APPLICATION
## published in accordance with Art. 158(3) EPC

(21) Application number: **93908094.1**

(22) Date of filing: **15.04.93**

(86) International application number:
**PCT/JP93/00483**

(87) International publication number:
**WO 93/21193 (28.10.93 93/26)**

(51) Int. Cl.6: **C07F 15/00**, A61K 31/72, C08B 37/08

(30) Priority: **17.04.92 JP 97744/92**

(43) Date of publication of application:
**01.02.95 Bulletin 95/05**

(84) Designated Contracting States:
**AT CH DE DK ES FR GB IT LI SE**

(71) Applicant: **SEIKAGAKU CORPORATION**
**1-5, Nihonbashi-honcho 2-chome**
**Chuo-ku,**
**Tokyo 103 (JP)**

(72) Inventor: **SAKURAI, Katsukiyo**
**527-6, Zohshiki 2-chome**
**Higashiyamato-shi,**
**Tokyo 207 (JP)**
Inventor: **MAEDA, Mitsuaki**
**5-28, Higashigaoka 2-chome**
**Meguro-ku,**
**Tokyo 152 (JP)**

(74) Representative: **Hansen, Bernd, Dr.**
**Dipl.-Chem. et al**
**Hoffmann, Eitle & Partner,**
**Patentanwälte,**
**Arabellastrasse 4**
**D-81925 München (DE)**

(54) **PLATINUM COMPLEX AND ANTINEOPLASTIC AGENT.**

(57) A platinum complex comprising a cycloalkanediamine platinum (II) and, coordinated thereto, the carboxyl and/or sulfate groups of chondroitin sulfate mainly comprising the 4-sulfate. It is electrophoretically uniform, contains at most 5 wt. % of platinum, and has a solubility of 10 mg/ml or above in water at 20 °C. This complex is remarkably improved in water solubility and antineoplastic activity and has a high organ selectivity for the liver and the like.

Rank Xerox (UK) Business Services
(3.10/3.09/3.3.4)

# Fig. 1

[Technical field]

The present invention relates to a platinum complex useful as an antitumor agent and an antitumor agent containing the platinum complex as an active ingredient.

[Technical background]

As a representative platinum complex having an antitumor activity, cisplatin has been known (Nature, 222, 385 (1969)). Cisplatin has an excellent antitumor activity against solid tumor, but it has a problem that side effects such as nephrotoxicity, etc. are strong.

In order to solve such a problem, platinum complexes having various leaving ligands have been synthesized. Also, compounds having saccharic acid such as glucuronic acid, etc. as said leaving ligand have been known (Japanese Provisional Patent Publication No. 44620/1979, Japanese Patent Publication No. 7194/1988 and Japanese Patent Publication No. 66318/1991). Further, a cisplatinum (1,2-cyclohexanediamine) complexed with chondroitin sulfate has been known (Japanese Provisional Patent Publication No. 116221/1984). However, in these compounds, even though side effects are alleviated, antitumor activities are insufficient or side effects are not alleviated sufficiently.

The present inventors synthesized platinum complexes having acidic polysaccharides such as chondroitin sulfate, hyaluronic acid, etc. as leaving ligands (carriers), and found that they had antitumor activities (Japanese Provisional Patent Publication No. 135201/1990).

Among them, the platinum complexes having chondroitin sulfate as a leaving ligand use chondroitin sulfate (containing chondroitin-6-sulfate as a main component) obtained from cartilages of a shark, and have platinum contents of about 20 % (w/w) or more and low solubilities in water (1 mg/ml or less). Further, these platinum complexes have low antitumor effects, low organ selectivities, high release rates of the platinum complexes into physiological saline (it is estimated that half-times in blood are short) and potent toxicities as compared with the platinum complexes having hyaluronic acid as a leaving ligand.

On the other hand, as to the platinum complex disclosed in Japanese Provisional Patent Publication No. 116221/1984 described above, derivation of chondroitin sulfate to be used is not described, its platinum content is as high as about 10 to 16 %, and the antitumor effect of said platinum complex is not described at all.

The present invention has been made for the purpose of solving the above problems of conventional platinum complexes having, as a leaving ligand, acidic polysaccharide, particularly chondroitin sulfate containing chondroitin-6-sulfate as a main component.

[Disclosure of the invention]

The present invention is to provide a platinum complex which comprises a cycloalkanediamine platinum (II) residue represented by the formula (1):

$$PtR^1 \quad (1)$$

[wherein $R^1$ represents a cycloalkanediamine having 3 to 8 carbon atoms] which is coordinated with a carboxyl group and/or a sulfuric acid group of chondroitin sulfate containing chondroitin-4-sulfate as a main component, which is electrophoretically uniform and has a platinum content of 5 % by weight or less and a solubility in water at 20 °C of 10 mg/ml or more.

Also, the present invention is to provide an antitumor agent comprising the above platinum complex as an active ingredient.

In the following, the present invention is explained in detail.

The above platinum complex of the present invention can be prepared by reacting a diaqua form of cycloalkanekadiamine platinum (II) obtained from cis-dihalogeno-cis-cycloalkanediamine platinum (II), cis-dihalogeno-trans-cycloalkandiamine platinum (II) or a mixture of them (hereinafter they are generally called cis-dihalogenocycloalkanediamine platinum (II)) represented by the formula (2):

$$X_2PtR^1 \quad (2)$$

[wherein $R^1$ has the same meaning as defined above and X represents a halogen atom], with chondroitin sulfate containing chondroitin-4-sulfate as a main component or an alkali salt thereof in an aqueous medium such as water, etc. under a light-shielding condition (e.g. in a dark room) and a slightly acidic to neutral condition (e.g. pH 4.0 to 7.0) at a reaction temperature of 0 to 40 °C (e.g. about room temperature) for several hours to several 10 days.

In the above formula (2), the cycloalkanediamine having 3 to 8 carbon atoms represented by $R^1$ may be exemplified by 1,2-cyclopentanediamine, 1,2-cyclohexanediamine, 1,2-cycloheptanediamine, 1,2-cyclooctanediamine, 1-amino-1-aminomethylcyclopentane, 1-amino-1-aminomethylcyclohexane, 1-amino-2-aminomethylcyclohexane, etc. and the halogen atom represented by X may be exemplified by chlorine, bromine, fluorine, etc.

The diaqua form of the cycloalkanediamine platinum (II) which is a starting material of the above reaction can be obtained by, for example, a conventional method in which an aqueous solution or an aqueous suspension of the above cis-dihalogenocycloalkanediamine platinum (II) is reacted with silver nitrate, silver sulfate, etc. preferably under a light-shielding condition to effect nitration (nitratization) or sulfation (sulfatization) and hydration, and precipitates formed (silver halide) are removed (Japanese Provisional Patent Publication No. 6290/1989; International Provisional Patent Publication No. 500745/1990; J. Am. Chem. Soc., 99, 120 (1973); and Chem. Biol. Interactions., 26, 227 (1979).

In place of using the diaqua form obtained by nitrating or sulfating the cis-dihalogenocycloalkanediamine platinum (II) in an aqueous medium as described above as a starting material, a compound obtained by contacting cis-dinitratocycloalkanediamine platinum (II) with an anion exchange resin (OH type) to substitute nitrato by a hydroxyl group may be used (see, for example, Japanese Patent Publication No. 51918/1990 and Japanese Patent Publication No. 58358/1991).

The chondroitin sulfate containing chondroitin-4-sulfate as a main component or an alkali salt thereof which is the other starting material is extracted mainly from cartilages (nasal cartilages, tracheal cartilages, etc.) of mammals such as a whale, a bovine, a horse, a sheep, a pig, etc. and purified according to a conventional method. In the present invention, the chondroitin sulfate refers to chondroitin sulfate containing 50 % or more of chondroitin-4-sulfate. The chondroitin sulfate containing chondroitin-4-sulfate (also referred to chondroitin sulfate A) as a main component, which is a constitutional component of the platinum complex of the present invention is clearly distinguished from chondroitin sulfate (prepared mainly from cartilages of teleosts such as a shark, a ray, etc.) containing chondroitin-6-sulfate (also referred to chondroitin sulfate C) as a main component, which is a starting material of the platinum complex disclosed in the above Japanese Provisional Patent Publication No. 135201/1990. The chondroitin sulfate containing chondroitin-4-sulfate as a main component (hereinafter referred to as chondroitin-4-sulfate) to be used in the present invention is preferably an alkali metal salt such as a sodium salt, a potassium salt, a lithium salt, etc.

The above reaction is carried out generally by using the chondroitin-4-sulfate in an excessive amount based on the diaqua form of the cycloalkanediamine platinum (II) so as to make the platinum content in the platinum complex which is a reaction product 5 % by weight or less.

After the reaction, the platinum complex of the present invention in a solution state can be recovered by removing an unreacted starting compound by means of membrane filtration, dialysis to water, etc., further removing a fraction insoluble in water from the reaction product according to a conventional method and, if necessary, carrying out fractionation in accordance with solubility in water. The platinum complex of the present invention in a powder state can be also recovered by treating this platinum complex solution according to a conventional method used when water-soluble polysaccharides are powdered such as lyophilization, drying under reduced pressure, spray drying, a precipitation operation with an organic solvent (alcohol, acetone, etc.) or the like.

The platinum complex of the present invention thus obtained is a complex wherein the cycloalkanediamine platinum (II) residue represented by the above formula (1) is coordinated with a carboxyl group and/or a sulfuric acid group of the chondroitin-4-sulfate, which is electrophoretically uniform and has a platinum content in the platinum complex of 5 % by weight or less, preferably 0.3 to 5 % by weight (about 0.005 to 0.1 or so of platinum atom per repeating unit of the chondroitin-4-sulfate), a chondroitin sulfate content of 80 to 96 % by weight, a solubility in water at 20 °C of 10 mg/ml or more, preferably 10 to 40 mg/ml and the following physical property values.

Average molecular weight: 5000 to 100000

Number of repeating disaccharide units per one platinum molecule: about 10 to 200

Specific rotation $[\alpha]_D$ (C = 1, $H_2O$): -44 to -10

Infrared absorption spectrum (IR): 3300 $cm^{-1}$ ($vNH_2$)

Ultraviolet absorption spectrum (UV): the local maximum absorption at 250 nm and 300 nm

Nuclear magnetic resonance spectrum (NMR): described below

Solubilities in various solvents: soluble in water, hardly soluble in methanol, ethanol and ethers.

Color of product: the color of the lyophilized product is white to pale yellow.

Release rate of platinum: the half-time of platinum in a physiological isotonic solution is 3 to 48 hours.

The platinum complex of the present invention is used as an antitumor agent for treating tumors such as various cancers, etc. preferably by parenteral administration to mammals including a human. As an agent for parenteral administration, there may be mentioned an aqueous agent, a suspension, an emulsion, etc. for intravenous administration, intramuscular administration, subcutaneous administration, intraperitoneal administration, local administration, etc. For example, the platinum complex of the present invention is dissolved or suspended in a solution for injection (distilled water for injection, a glycerin aqueous solution, a propylene glycol aqueous solution, etc.), if necessary, with a pH adjusting agent (hydrochloric acid, sodium hydroxide, lactic acid, sodium lactate, sodium monohydrogen phosphate, sodium dihydrogen phosphate, etc.) and an isotonizing agent (sodium chloride, glucose, etc.), and the resulting solution or suspension is subjected to sterile filtration and charged into an ampule as an aqueous agent. Alternatively, mannitol, dextrin, cyclodextrin or the like is added to the platinum complex of the present invention, if necessary, and the mixture is charged into an ampule or a vial as powder (a lyophilized product, crystals, fine crystals, etc.) to prepare an injection which is dissolved when it is used. Further, an emulsifier (lecithin, polysorbate 80, polyoxyethylene hardened castor oil, etc.) is added to the platinum complex of the present invention to emulsify said complex in water, whereby an emulsion for injection is prepared.

The dose of the above antitumor agent varies depending on age, sex and disease conditions of a patient, but it is generally about 0.01 mg to 10 mg in terms of a platinum amount per day and per 1 kg of body weight and the antitumor agent is desirably administered at one time or in several divided doses.

[Brief description of the drawings]

Fig. 1 shows results of electrophoresis of the platinum complexes of the present invention and Comparative example. As described below, in the figure, 1 is an electrophoretic image of Diaqua form DACHP, 2 is that of CSA, and 3 to 7 are those of CSA-DACHP 1 to 5.

Fig. 2 shows high performance liquid chromatography of CSA.

Fig. 3 shows high performance liquid chromatography of Diaqua form DACHP.

Fig. 4 shows high performance liquid chromatography of CSA-DACHP 1.

Fig. 5 shows high performance liquid chromatography of CSA-DACHP 2.

Fig. 6 shows examination results of antitumor effects of the platinum complexes of the present invention and Comparative example.

Fig. 7 shows examination results of antitumor effects of the platinum complexes of Comparative example 2.

Fig. 8 shows kinetics *in vivo* of the platinum complex of the present invention.

[Best mode for practicing the present invention]

In the following, the present invention is described specifically by referring to Examples showing preparation examples of the platinum complexes of the present invention, Comparative examples showing preparation examples of control platinum complexes and Test examples showing antitumor effects. However, these examples are illustrative and the scope of the present invention is not limited thereby.

Reference example 1

In 80 ml of water was dissolved 5.5 g of (±)trans-1,2-cyclohexanediamine, to this aqueous solution was added a platinous potassium chloride aqueous solution (20 g/300 ml), and the mixture was reacted at room temperature in a dark room for 20 hours. Yellow precipitates formed were collected by filtration, washed sufficiently with water and dried. The precipitates were cis-dichloro-(±)trans-(1,2-cyclohexanediamine)-platinum (II) (hereinafter referred to as $DACHPCl_2$), and the yield was 17.4 g.

In 50 ml of water was suspended 8.7 g of $DACHPCl_2$, a silver nitrate aqueous solution (7.784 g/80 ml) was added thereto, and the mixture was reacted at room temperature in a dark room for 20 hours. Precipitates of silver chloride formed were removed to prepare 155 ml (56 mg/ml) of a diaqua form of (±)-trans-(1,2-cyclohexanediamine)platinum (II) (DACHP) (hereinafter referred to as Diaqua form DACHP).

Example 1

In 100 ml of water was dissolved 500 mg of sodium chondroitin-4-sulfate derived from cartilages of mammals (whale) (containing about 75 % of chondroitin-4-sulfate, average molecular weight of 30000; produced by Seikagaku Kogyo Kabushiki Kaisha) (hereinafter referred to as CSA), and 0.446 ml (containing 25 mg) of Diaqua form DACHP prepared in Reference example 1 was added thereto. The mixture was adjusted to pH 6.0 with a 0.1N-sodium hydroxide aqueous solution and then reacted at 5 °C in a dark room for 20 hours. The reaction mixture was subjected to dialysis to distilled water to remove an unreacted reaction reagent having a low molecular weight and by-products. The resulting product was filtered with a filtration film having a pore size of 0.2 $\mu$m and then lyophilized to obtain 554 mg of the platinum complex of the present invention (lot name; CSA-DACHP 1).

Lot: CSA-DACHP 1

Yield: 554 mg

Content of chondroitin-4-sulfate: 95.68 %

Content of platinum: 1.03 %

Number of repeating disaccharide units per one platinum molecule: 38.18

Solubility: 22.2 mg/ml (20 °C, in water)

Color of product: white to pale yellow

The solubility of the platinum complex was measured by adding purified water to the sample, stirring the mixture at 20 °C for 20 hours, subjecting the mixture to centrifugation (3000 rpm) for 5 minutes and then quantitating the chondroitin sulfate content of a supernatant by a carbazole-sulfuric acid method.

Example 2 and Comparative example 1

According to Example 1, 0.223 ml (12.5 mg), 0.893 ml (50 mg), 1.786 ml (100 mg), 2.679 ml (150 mg) or 3.571 ml (200 mg) of Diaqua form DACHP prepared in Reference example 1 was added to each 500 mg of a CSA aqueous solution, and the mixtures were reacted to prepare platinum complexes having different platinum contents and solubilities in water (lot names; CSA-DACHP 0, CSA-DACHP 2, CSA-DACHP 3, CSA-DACHP 4 and CSA-DACHP 5, respectively). The yields and analytical values of the respective platinum complexes are shown in Table 1.

Table 1

| Lot | Yield (mg) | CSA content (%) | Platinum content (%) | Solubility (mg/ml $H_2O$) |
|---|---|---|---|---|
| (Example 2) | | | | |
| CSA-DACHP 0 | 502 | 97.47 | 0.51 | 28.7 |
| CSA-DACHP 2 | 533 | 90.08 | 1.80 | 10.5 |
| CSA-DACHP 3 | 585 | 85.55 | 3.96 | 10.3 |
| (Comparative example 1) | | | | |
| CSA-DACHP 4 | 620 | 81.03 | 5.83 | 3.9 |
| CSA-DACHP 5 | 651 | 75.58 | 8.36 | 2.1 |

Physical property values

When the physical property values of the respective CSA-DACHP samples obtained in the above Examples and Comparative example 1 were measured, the following results were obtained.

1. Electrophoresis

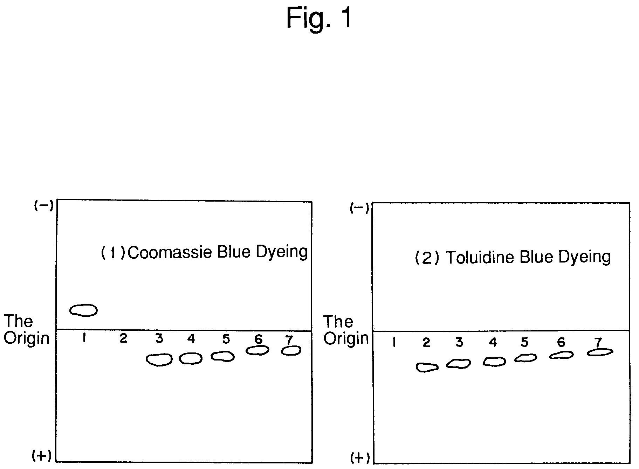

CSA-DACHP 1, 2, 3, 4 and 5 were dissolved in water so as to have concentrations of 20, 10, 5, 4 and 4 mg/ml, respectively, and 8 $\mu$l of the respective solutions were coated on a cellulose acetate film (SEPARAX, Jyoko K.K.) for electrophoresis. As a control, CSA was dissolved in water so as to have a concentration of 1.04 mg/ml, Diaqua form DACHP was dissolved in water so as to have a concentration of 1.098 mg/ml, and 2 $\mu$l and 8 $\mu$l of the respective solutions were coated on the above film. Electrophoresis was carried out under the following conditions. The results are shown in Fig. 1. In the figure, 1 is an electrophoretic image of Diaqua form DACHP, 2 is that of CSA, and 3 to 7 are those of CSA-DACHP 1 to 5, respectively.

Electrophoresis conditions: 0.5 mA/cm, 15 minutes, 0.1M pyridine/formic acid (pH 3.0)

Dyes:  (1) Coomassie Blue dyeing (dyeing for platinum)

(2) Toluidine Blue dyeing (dyeing for saccharide)

## 2. High performance liquid chromatography

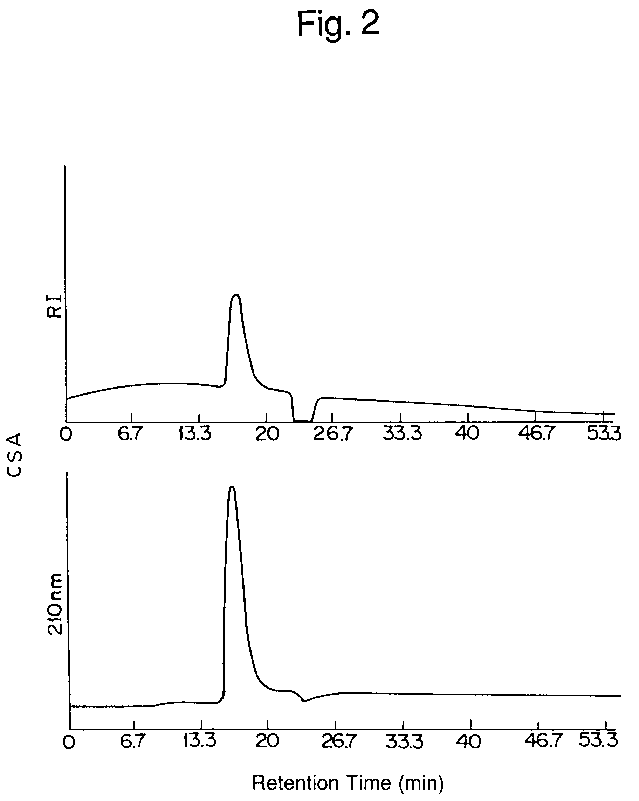

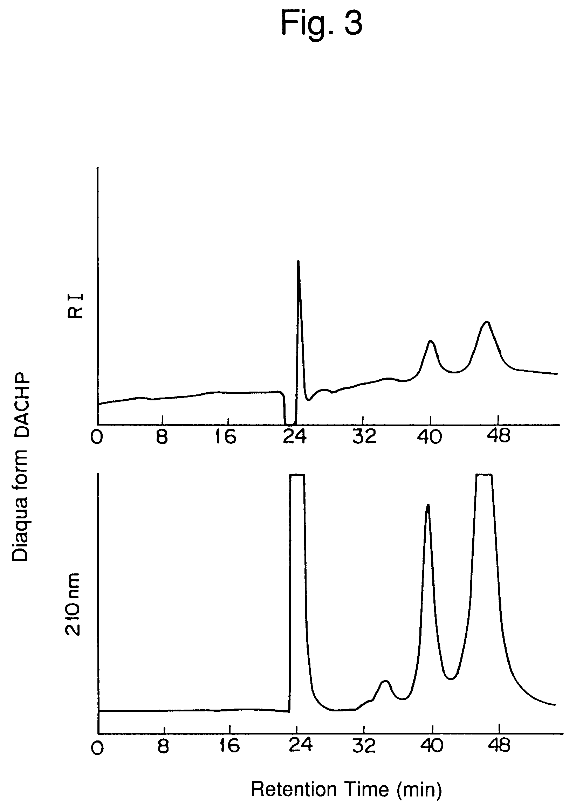

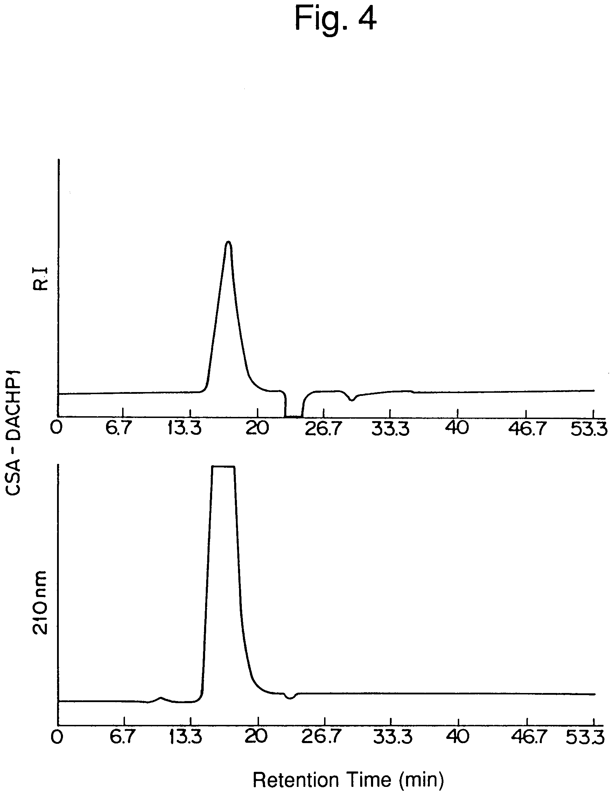

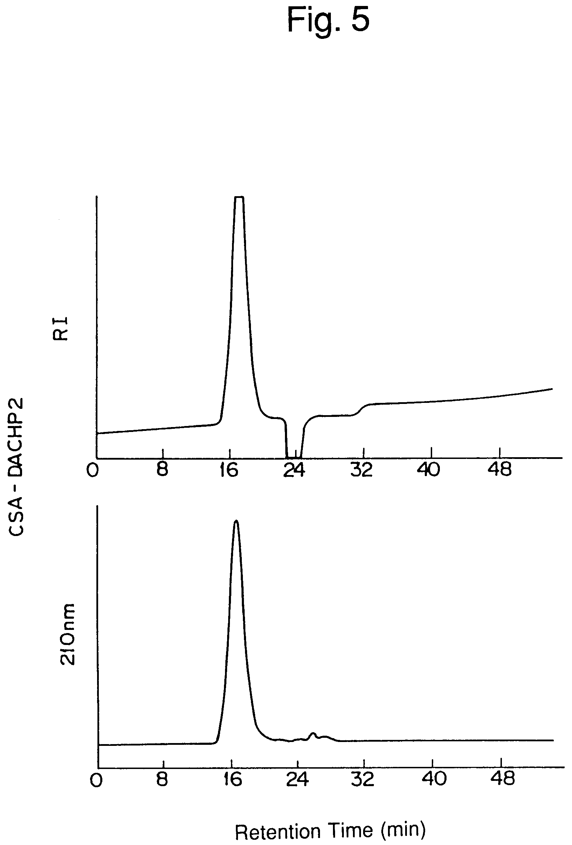

The respective specimens of CSA-DACHP 1 and 2 were dissolved in water so as to have a concentration of 1 mg/ml, CSA was dissolved in water so as to have a concentration of 0.2 mg/ml, and Diaqua form DACHP was dissolved in water so as to have a concentration of 0.28 mg/ml, respectively. Each 20 $\mu$l was charged into a column for high performance liquid chromatography. As the column, TSKgel-G4000PW$_{XL}$ manufactured by Toso K.K. was used, a solvent was a 0.1M sodium chloride solution, a flow rate was 0.5 ml/min, and detection was carried out by measuring differential refraction (RI) and absorption at a wavelength of 210 nm. The results are shown in Fig. 2 to Fig. 5.

## 3. Ultraviolet absorption spectrum (UV)

The respective CSA-DACHP specimens had the local maximum absorptions at 250 and 300 nm, and as the platinum amount was larger, their absorption degrees were larger.

## 4. Infrared absorption spectrum (IR)

When IR was measured for the respective specimens of CSA-DACHP 0, 1, 2 and 3, absorption of vNH$_2$ was observed at 3300 cm$^{-1}$. As the platinum amount was larger, the absorption was larger.

## 5. Specific rotation [$\alpha$]$_D$

The results of measuring specific rotations of the platinum complexes of the above respective Examples and Comparative examples (Comparative example 2 is described below) are shown in Table 2.

Table 2

|  | Lot | [$\alpha$]$_D$ (C = 1, H$_2$O) |
|---|---|---|
| Example 1<br>Example 2<br>Example 2 | CSA-DACHP 1<br>CSA-DACHP 2<br>CSA-DACHP 3 | -38.7<br>-36.8<br>-32.0 |
| Comparative example 1 | CSA-DACHP 4<br>CSA-DACHP 5 | -28.2<br>-21.8 |
| Comparative example 2 | CSC-DACHP 1<br>CSC-DACHP 2<br>CSC-DACHP 3<br>CSC-DACHP 4<br>CSC-DACHP 5 | -20.4<br>-18.4<br>-15.6<br>-12.1<br>-10.6 |

## 6. Nuclear magnetic resonance spectrum (NMR)

It was difficult to find a clear difference between NMR of the platinum complex containing the chondroitin-4-sulfate having a molecular weight of several tens of thousands and that of the chondroitin-4-sulfate which is a starting material. Therefore, by using chondroitin-4-sulfate oligosaccharide (containing 75 % of unsaturated disaccharide and 25 % of unsaturated tetrasaccharide) obtained by degrading the same sodium chondroitin-4-sulfate as that used in Examples with an enzyme, a platinum complex was prepared according to a method of Reference example 2 described below, and NMR of the chondroitin-4-sulfate oligosaccharide and that of the platinum complex were compared.

As a result, in the chondroitin-4-sulfate oligosaccharide, a proton bonded to a double bond was detected at 6 ppm, but in the platinum complex, this proton was shifted and disappeared from this position. From the ratio of 10H (cyclohexane) at 1.2 to 2.6 ppm to 3H (CH$_3$CO-NH-) at 2 ppm of the platinum complex, the ratio of the chondroitin-4-sulfate oligosaccharide to Diaqua form DACHP was estimated to be 1:1.

Further, from this result, it is estimated that a carboxyl group or a sulfuric acid group of the chondroitin-4-sulfate participates in bonding of the chondroitin-4-sulfate and platinum.

Reference example 2

1. Preparation of chondroitin-4-sulfate oligosaccharide

An aqueous solution (12 g/150 ml, adjusted to pH 7.5 with a sodium hydroxide aqueous solution) of chondroitin-4-sulfate (MW 30000) derived from whale cartilages was degraded at 37 °C for 220 minutes by adding 1000 units/ml of a commercially available Chondroitinase ABC (derived from *Proteus vulgaris*; Seikagaku Kogyo Kabushiki Kaisha). The reaction mixture was filtered with a filtration film having a pore size of 0.22 $\mu$m and then lyophilized to obtain 10.7 g of chondroitin-4-sulfate oligosaccharide.

2. Preparation of platinum complex of chondroitin-4-sulfate oligosaccharide

In 50 ml of water was dissolved 500 mg of the chondroitin-4-sulfate oligosaccharide, 6.786 ml (380 mg) of a Diaqua form DACHP aqueous solution (56 mg/ml) was added thereto, and the mixture was reacted at 5 °C for 20 hours. The reaction mixture was desalted with a ultrafiltration membrane having a fractionation molecular weight of 500 and lyophilized to obtain 650 mg of a platinum complex of the chondroitin-4-sulfate oligosaccharide. The platinum content of this platinum complex was 25.9 %, and by IR analysis, absorption of vNH$_2$ was observed at 3300 cm$^{-1}$.

Comparative example 2

By using sodium chondroitin-6-sulfate derived from whale cartilages (containing about 75 % of chondroitin-6-sulfate, average molecular weight of 30000; produced by Seikagaku Kogyo Kabushiki Kaisha) (hereinafter referred to as CSC) and Diaqua form DACHP prepared in Reference example 1, platinum complexes were prepared in the same manner as in Example 1. Their yields and analytical values are shown in Table 3.

Table 3

| Lot | Yield (mg) | CSC content (%) | Platinum content (%) | Solubility (mg/ml H$_2$O) |
|---|---|---|---|---|
| CSC-DACHP 1 | 519 | 92.81 | 1.24 | 21.1 |
| CSC-DACHP 2 | 537 | 85.43 | 2.54 | 10.6 |
| CSC-DACHP 3 | 550 | 81.47 | 5.01 | 2.24 |
| CSC-DACHP 4 | 442 | 82.32 | 6.68 | 2.13 |
| CSC-DACHP 5 | 464 | 76.99 | 8.94 | 2.15 |

Test example 1 (antitumor effect)

To an abdominal cavity of a CDF mouse in which 1 x 10$^5$ of L1210 cells had been previously transplanted, a solution to be tested in which each platinum complex or DACHPCl$_2$ described above was dissolved in physiological saline so as to have a predetermined concentration was administered on the first day and the fifth day after the transplantation, and each prolonged time of life and prolongation rate were examined. The results are shown in Table 4. As to a control, only physiological saline was administered in place of the solution to be tested. Also, the antitumor effects of platinum complexes (CS-t-DACHP, CS-m-DACHP) which were the most similar to the platinum complex of the present invention, disclosed in Japanese Provisional Patent Publication No. 135201/1990, which were measured in the same manner as described above are shown in Table 5.

Table 4

| Lot | Dose | | Prolonged time of life (day) | T/C (%) | Mouse of which life is prolonged for 60 days/all mice |
|---|---|---|---|---|---|
| | ($\mu$mol Pt/kg) | (mg Pt/kg) | | | |
| (Examples) | | | | | |
| CSA-DACHP 0 | 4.80 | 0.94 | >60 | >750 | 5/6 |
| CSA-DACHP 1 | 4.80 | 0.94 | >60 | >750 | 5/6 |
| CSA-DACHP 2 | 4.80 | 0.94 | >45 | >563 | 3/6 |
| CSA-DACHP 3 | 4.80 | 0.94 | >46 | >575 | 3/6 |
| (Comparative examples) | | | | | |
| CSA-DACHP 4 | 4.80 | 0.94 | 20.5 | 256 | 1/6 |
| CSA-DACHP 5 | 4.80 | 0.94 | 19.0 | 238 | 1/6 |
| DACHPCl$_2$ | 13.70 | 2.67 | 19.5 | 244 | 1/6 |
| | 34.20 | 6.67 | 9.0 | 110 | 0/6 |
| Control | | | 8.0 | 100 | 0/6 |

T/C (%) represents the ratio of the prolonged time of life of the group to which the solution to be tested was administered to the prolonged time of life of the control group.

Table 5

| Japanese Provisional Patent Publication No. 135201/1990 (platinum complexes of Examples 1 and 2, extracted from Table of Example 10) | | | | | |
|---|---|---|---|---|---|
| Lot | Dose | | Prolonged time of life (day) | T/C (%) | Mouse of which life is prolonged for 60 days/all mice |
| | ($\mu$mol Pt/kg) | (mg Pt/kg) | | | |
| CS-t-DACHP | 2.05 | 0.40 | >21.5 | >235 | 1/6 |
| | 5.69 | 1.11 | >38.8 | >425 | 3/6 |
| | 28.55 | 5.57 | >15.7 | >172 | 1/6 |
| CS-m-DACHP | 18.86 | 3.68 | >20.5 | >224 | 2/6 |

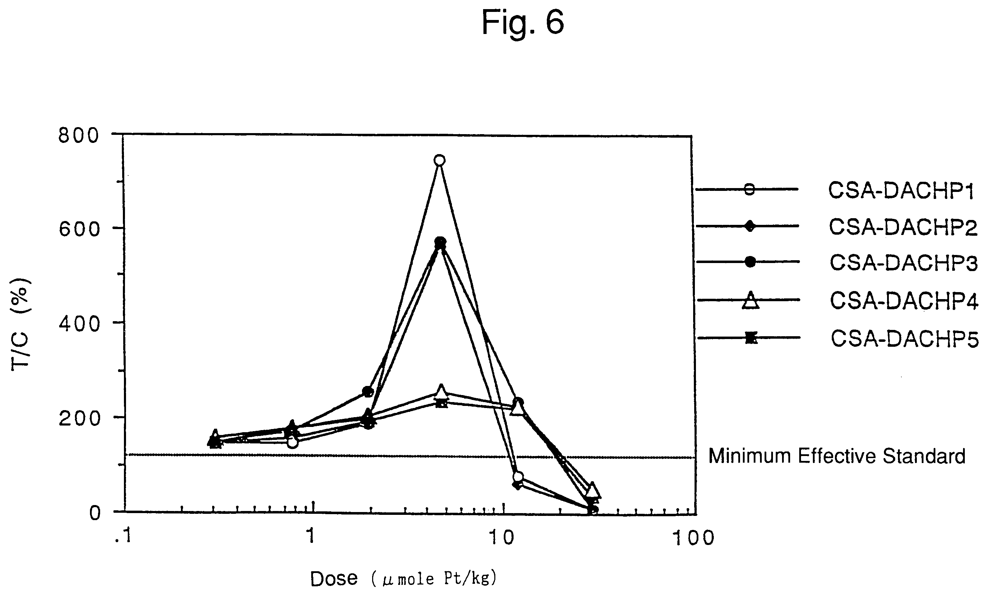

By changing the doses of the above platinum complexes CSA-DACHP 1 to 5, antitumor effects were examined in the same manner as described above. The results are shown as T/C (%) in Fig. 6.

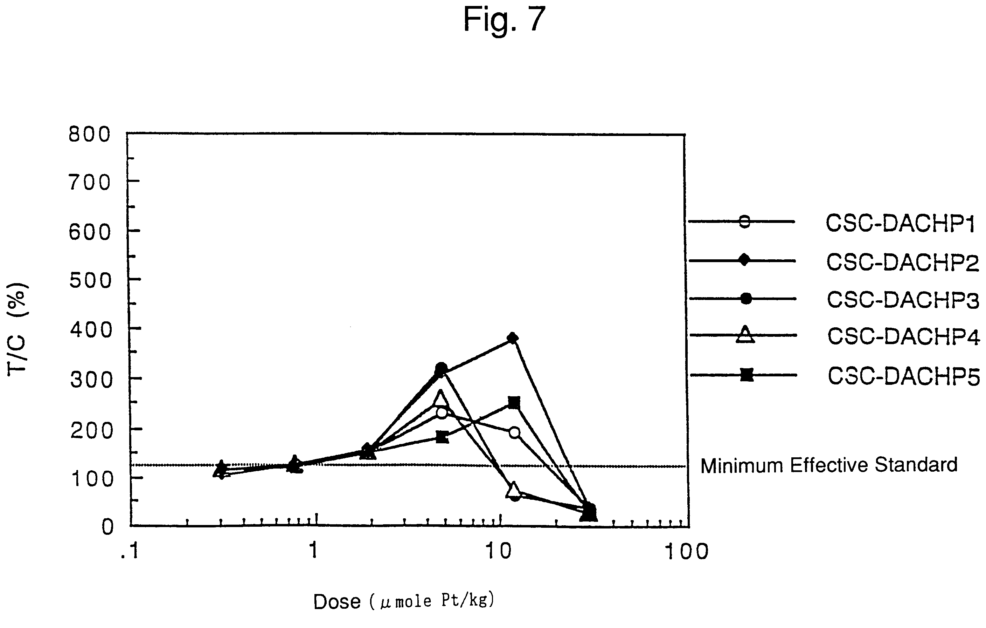

On the other hand, for CSC-DACHP 1 to 5 of Comparative example 2, antitumor effects were examined in the same manner. The results are shown in Fig. 7.

To a mouse in which sarcoma 180 cells which were solid tumor had been previously transplanted, the platinum complex of the present invention was administered intra-peritoneally in the same manner as described above, and its antitumor effect was examined. The platinum complex of the present invention also exhibited an excellent antitumor effect on solid tumor.

Test example 2 Kinetics of CSA-DACHP *in vivo* (organ selectivity)

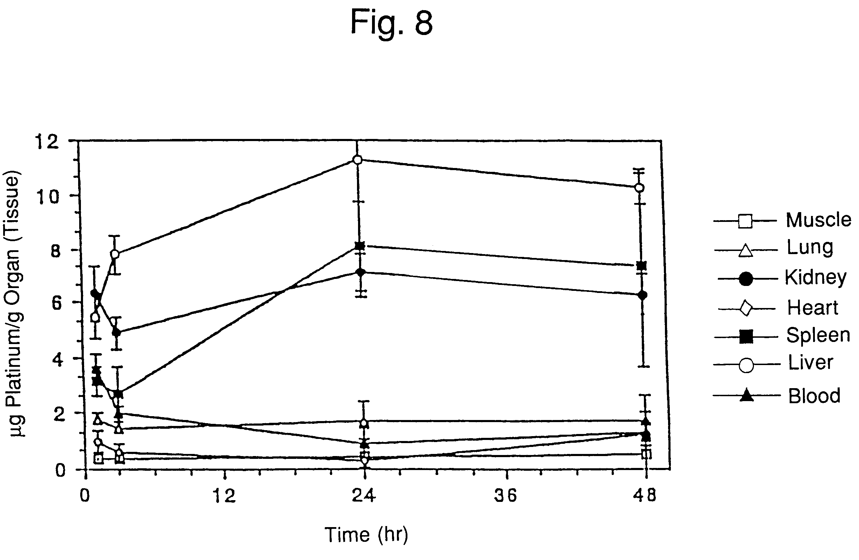

To an abdominal cavity of an ICR mouse, 32.5 mg/kg of CSA-DACHP 1 was administered, and changes with a lapse of time of distributions of platinum to the respective organs were measured. The results are shown in Fig. 8.

As shown in Fig. 8, it was observed that a large amount of platinum was distributed to a liver. From this result, it was suggested that particularly a tumor of a liver was inhibited specifically.

Example 3

According to Example 1, to an aqueous solution of 500 mg of sodium chondroitin-4-sulfate derived from cartilages of mammals (whale) (containing about 75 % of chondroitin-4-sulfate, average molecular weight of 22000: produced by Seikagaku Kogyo Kabushiki Kaisha) (hereinafter referred to as CSA (W22000)) and an aqueous solution of 500 mg of sodium chondroitin-4-sulfate derived from tracheal cartilages of mammals (cattle) (containing about 60 % of chondroitin-4-sulfate, average molecular weight of 20000: produced by Seikagaku Kogyo Kabushiki Kaisha) (hereinafter referred to as CSA (BT20000)), 0.257 ml (14.4 mg) of Diaqua form DACHP prepared in Reference example 1 was added, respectively, and the mixtures were reacted to prepare platinum complexes of lots CSA (W22000)-DACHP 6 and CSA (BT20000)-DACHP 7. The yields and analysis tables of the respective platinum complexes are shown in Table 6.

Table 6

| Lot | Yield (mg) | CSA content (%) | Platinum (%) | content Solubility (mg/ml $H_2O$) |
|---|---|---|---|---|
| CSA (W22000)-DACHP 6 | 503 | 97.5 | 0.93 | 30.6 |
| CSA (BT20000)-DACHP 7 | 501 | 97.3 | 0.93 | 28.6 |

Test example 3 (antitumor effect)

According to Test example 1, the lots prepared in Example 3 were tested. The results are shown in Table 7.

Table 7

| Lot | Dose | | Prolonged time of life (day) | T/C (%) | Mouse of which life is prolonged for 60 days/all mice |
|---|---|---|---|---|---|
| | (μmol Pt/kg) | (mg Pt/kg) | | | |
| CSA (W22000)-DACHP 6 | 12 | 2.34 | >60 | >750 | 4/6 |
| CSA (BT20000)-DACHP 7 | 12 | 2.34 | >60 | >750 | 5/6 |

[Industrial applicability]

According to the present invention, in the platinum complex prepared by selecting specific chondroitin-4-sulfate which has not been used in a conventional platinum complex having an acidic polysaccharide as a leaving ligand (carrier) and controlling a platinum content to 5 % by weight or less, solubility in water is improved to a great extent and as a result, an antitumor activity can be improved.

Also, the platinum complex of the present invention has high organ selectivity to a liver, a spleen, etc. and a large amount thereof is distributed particularly to a liver so that it is useful as an antitumor agent for specifically inhibiting tumors of these organs. Further, the release rate of platinum from the platinum complex is slow so that it is a controlled releasing antitumor agent of which an antitumor effect can be maintained for a long time.

**Claims**

1. A platinum complex which comprises a cycloalkanediamine platinum (II) residue represented by the following formula:

$PtR^1$

10

[wherein R$^1$ represents a cycloalkanediamine having 3 to 8 carbon atoms] which is coordinated with a carboxyl group and/or a sulfuric acid group of chondroitin sulfate containing chondroitin-4-sulfate as a main component, which is electrophoretically uniform and has a platinum content of 5 % by weight or less and a solubility in water at 20 °C of 10 mg/ml or more.

2.  The platinum complex according to Claim 1, wherein R$^1$ is 1,2-cyclopentanediamine, 1,2-cyclohexanediamine, 1,2-cycloheptanediamine, 1,2-cyclooctanediamine, 1-amino-1-aminomethylcyclopentane, 1-amino-1-aminomethylcyclohexane or 1-amino-2-aminomethylcyclohexane.

3.  The platinum complex according to Claim 1, wherein the chondroitin sulfate containing chondroitin-4-sulfate as a main component is derived from mammals.

4.  The platinum complex according to Claim 1, wherein the chondroitin sulfate containing chondroitin-4-sulfate as a main component is derived from a whale or a bovine.

5.  The platinum complex according to Claim 1, wherein the chondroitin sulfate containing chondroitin-4-sulfate as a main component is an alkali metal salt.

6.  The platinum complex according to Claim 1, wherein the chondroitin sulfate containing chondroitin-4-sulfate as a main component is contained in an amount of 80 to 96 % by weight.

7.  The platinum complex according to Claim 1, wherein the platinum complex has a platinum content of 0.3 to 5 % by weight and a solubility in water at 20 °C of 10 to 40 mg/ml.

8.  The platinum complex according to Claim 1, wherein the platinum complex has an average molecular weight of 5,000 to 100,000, a repeating disaccharide unit number per one platinum molecule of about 10 to 200 and a specific rotation $[\alpha]_D$ (C = 1, H$_2$O) of -44 to -10.

9.  An antitumor agent comprising the platinum complex according to Claim 1 as an active ingredient.

# Fig. 1

(1) Coomassie Blue Dyeing

(2) Toluidine Blue Dyeing

# Fig. 2

# Fig. 3

# Fig. 4

# Fig. 5

Retention Time (min)

Fig. 6

CSA-DACHP1
CSA-DACHP2
CSA-DACHP3
CSA-DACHP4
CSA-DACHP5

Minimum Effective Standard

T/C (%)

Dose ($\mu$mole Pt/kg)

EP 0 636 631 A1

# Fig. 7

Fig. 8

International application No.

PCT/JP93/00483

| A. CLASSIFICATION OF SUBJECT MATTER |
|---|
| Int. Cl$^5$  C07F15/00, A61K31/72, C08B37/08 |

According to International Patent Classification (IPC) or to both national classification and IPC

| B. FIELDS SEARCHED |
|---|
| Minimum documentation searched (classification system followed by classification symbols) |
| Int. Cl$^5$  C07F15/00, A61K31/72, C08B37/08 |

Documentation searched other than minimum documentation to the extent that such documents are included in the fields searched

Electronic data base consulted during the international search (name of data base and, where practicable, search terms used)
  CAS ONLINE

## C. DOCUMENTS CONSIDERED TO BE RELEVANT

| Category* | Citation of document, with indication, where appropriate, of the relevant passages | Relevant to claim No. |
|---|---|---|
| A | JP, A, 4-82836 (Seikagaku Corp.), March 16, 1992 (16. 03. 92), & WO, A, 9201720 & EP, A, 493622 | 1-9 |
| A | JP, A, 4-80202 (Seikagaku Corp.), March 13, 1992 (13. 03. 92), & WO, A, 9201720 & EP, A, 493622 | 1-9 |
| A | JP, A, 4-80201 (Seikagaku Corp.), March 13, 1992 (13. 03. 92), & WO, A, 9201720 & EP, A, 493622 | 1-9 |
| A | JP, A, 2-135201 (Seikagaku Corp.), May 24, 1990 (24. 05. 90), (Family: none) | 1-9 |
| A | JP, A, 59-116221 (Inco Research & Development Center, Inc.), July 5, 1984 (05. 07. 84), & EP, A, 111388 & US, A, 4584392 & US, A, 4673754 | 1-9 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| July 13, 1993 (13. 07. 93) | August 3, 1993 (03. 08. 93) |

| Name and mailing address of the ISA/ | Authorized officer |
|---|---|
| Japanese Patent Office | |
| Facsimile No. | Telephone No. |

Form PCT/ISA/210 (second sheet) (July 1992)